# FASCICULE DE BREVET EUROPEEN

(11) **EP 0 643 960 B1**
(45) Date de publication et mention de la délivrance du brevet: **24.11.1999**
(21) Numéro de dépôt: 94401958.7
(22) Date de dépôt: 02.09.1994
(51) Int. Cl.: A61K 7/48

(54) **Utilisation pour le traitement des peaux mixtes d'une quantité efficace de substances actives**
Verwendung von wirksamen Mengen von aktiven Stoffen zur Behandlung von Mischhaut
Use of an effective quantitiy of active substances for the treatment of mixed skin

(30) Priorité: 21.09.1993 FR 9311240
(43) Date de publication de la demande: 22.03.1995
(73) Titulaire: LABORATOIRE DE BIOLOGIE VEGETALE YVES ROCHER, 56201 La Gacilly (FR)
(72) Inventeur: Khaiat, Alain, F-75016 Paris (FR)
(74) Mandataire: Le Guen, Gérard

(56) Documents cités:
- EP-A- 0 039 857
- EP-A- 0 117 613
- EP-A- 0 183 322
- EP-A- 0 281 812
- EP-A- 0 388 275
- EP-A- 0 414 605
- FR-A- 2 287 214
- FR-A- 2 305 172
- FR-A- 2 405 068
- FR-A- 2 648 347
- FR-A- 2 692 480
- PATENT ABSTRACTS OF JAPAN vol. 13, no. 491 (C-650) (3839) & JP-A-01 193 207 (NISSHIN OIL MILLS LTD)
- Soap, Perfumery & Cosmetics, London, GB, Vol 63, N 12, Decembre 1990, pages 41-45 part 2

## Description

La présente invention concerne l'utilisation pour le traitement esthétique des peaux mixtes d'une quantité efficace de substances actives.

En cosmétologie, on qualifie de "peau mixte" les peaux des personnes qui présentent une dualité entre deux parties de leur visage, c'est-à-dire qui présentent une zone grasse et une zone sèche.

Cette dualité est essentiellement due à la répartition des glandes sébacées. Ainsi, la zone grasse représentée par le front, le nez et le menton comprend 900 glandes sébacées au cm². La zone sèche représentée par les joues comprend seulement 400 glandes sébacées par cm².

La difficulté pour le cosmétologue réside dans la mise au point de produits devant s'adapter à cette combinaison de parties sèches et grasses de la peau, tout en améliorant son aspect général.

Les derniers produits connus pour le soin des peaux mixtes est le produit BIOPUR® DE Biotherm et le produit MODERACTIVE® de Jeanne Gatineau. Ces produits ont comme principale activité d'absorber l'excès de sébum et donc de traiter plus particulièrement les zones grasses. Le problème lié aux peaux mixtes n'a par conséquent pas été résolu par les produits cités.

Il est connu que les lipides cutanés de surface de la peau représentent un mélange de lipides synthétisés par deux sources : les glandes sébacées et l'épiderme.

Le rôle de ces lipides est passé de simple agent plastifiant à celui de barrière épidermique régissant les propriétés de rétention et de réception de l'eau, les différences de perméabilité d'agents appliqués localement, ainsi que la cohésion et la desquamation du stratum corneum.

3 % des lipides de surface proviennent de la sécrétion de l'épiderme, alors que 97 % sont dûs à l'excrétion par les glandes sébacées.

Par ailleurs, le rythme circadien, l'âge et le sexe de la personne ont une influence sur le taux d'excrétion des lipides par les glandes sébacées.

Ainsi, entre 13 et 20 ans, le taux d'excrétion par les glandes sébacées triple, pour ensuite diminuer progressivement.

Les glandes sébacées d'une personne de sexe féminin présentant une peau normale sécrètent de 0,75 à 1 µg/cm²/min sébum, alors que celles d'une personne à peau mixte sécrètent au niveau des zones grasses 50 % de sébum en plus du taux normal et au niveau des zones sèches ont une sécrétion qui diminue significativement.

De même, pour les personnes de sexe masculin, le taux de sécrétion d'une peau normale est de 1 à 1,5 µg/cm²/min, le taux de sécrétion pour les peaux mixtes étant au niveau des zones grasses augmenté de 65 %, et diminué significativement au niveau des zones sèches.

Il est un fait que le sébum, fluide excrété par les glandes sébacées, est la principale source des lipides cutanés de surface.

Or, le sébum comprend 31 % de triglycérides, 5 % de diglycérides, 24 % d'acides gras libres, 15 % de squalène et 25 % de cires.

Ainsi, deux faits se révèlent caractéristiques des "peaux mixtes".

Pour les zones sèches : la réduction significative des triglycérides, pouvant aller jusqu'à une disparition dans les cas de xérose les plus sévères.

Pour les zones grasses : l'importance quantitative des triglycérides. Ceux-ci ne restent malheureusement pas à l'état "natif", mais sont progressivement dégradés, sous l'action d'enzymes, en diglycérides et monoglycérides, et finalement en acides gras libres responsables de la plupart des troubles et défauts des peaux mixtes (aspect luisant et gras, surface le plus souvent irritée, peau sujette à une réponse inflammatoire).

Ces enzymes, appelées lipases, sont issues principalement de la flore cutanée : Propionibacterium acnes, Staphylococcus epidermidis, Propionibacterium granulosum.

A défaut de pouvoir imiter la production du sébum et donc d'agir sur les mécanismes hormonaux, les auteurs de la présente invention ont cherché à résoudre le problème des "peaux mixtes" en agissant à la fois sur les zones grasses et sèches.

La présente invention a donc pour objet l'utilisation pour le traitement esthétique des peaux mixtes d'une quantité efficace d'une seule et même substance ayant à la fois des propriétés émolliente et inhibitrice de lipases,choisie parmi l'huile de Limnanthes Alba (ou Shambrila) et l'huile de Jessenia Bataua (ou Hunguraghua).

Le terme "émollient" signifie que la substance a une activité bénéfique pour rétablir le manteau lipidique de surface et permettre à l'épiderme de garder toute sa souplesse et élasticité, ainsi que pour contribuer à rétablir le film hydrolipidique.

Ces substances à double activité ont été testées. L'huile de Shambrila s'est montrée particulièrement efficace pour le traitement des "peaux mixtes". En cosmétique, l'huile de Shambrila raffinée est particulièrement recherchée pour son toucher soyeux, son effet émollient et hydratant. Elle est extraite de semences de Limnanthes Alba, dont le caractère unique est d'être constitué à maturité d'un taux très important de triglycérides formés d'acides gras à très longues chaînes (C₂₀-C₂₂). L'huile contient 96 % d'acides gras à chaîne longue de plus de 20 carbones, dont 18 % d'acides gras polyinsaturés.

La méthode utilisée pour évaluer la quantité nécessaire des substances inhibitrices des lipases à utiliser dans les compositions cosmétiques repose sur l'utilisation d'un appareil simple. On maintient le pH et la température d' un mélange huile d'olive-eau, auquel on ajoute une enzyme ayant des propriétés comparables aux lipases de la flore cutanée, que l'on met au bain-marie.

Ainsi, on prépare le mélange qui constitue le substrat en mélangeant 12 ml d'huile d'olive, 15 ml d'azoture de Na (0,1 % aqueux), 7,5 ml d'eau distillée, 3 ml de substance à tester et 3 ml de tampon TRIS pour obtenir un pH 8. On ajoute alors 3 ml d'une solution aqueuse à 5 % de lipase de Rhizopus arrihzus.

On trace alors des courbes qui permettent de comparer les substances antilipase entre elles et d'évaluer leur activité en fonction de leur concentration dans le milieu réactionnel.

Les substances actives présentant les propriétés inhibitrices de lipases agissent par des phénomènes d'encombrement stérique en bloquant le site actif de l'enzyme.

On a donc déterminé les concentrations efficaces des substances anti-lipase dans les compositions de la présente invention :

| | |
|---|---|
| Huile de Limnanthes Alba | 0,5 à 15 % |
| Jessenia Bataua | 0,5 à 15 %. |

Des tests ont ensuite été réalisés en mesurant le taux de sébum sur la peau avant et après deux semaines d'application du (ou des) produit identifié comme actif en tant qu'inhibiteur de lipases et émollient. Des émulsions nettoyantes, lotions et crèmes ont été testées en fonction du pourcentage en poids des substances actives. Dans ces essais, on a utilisé comme composition selon l'invention les compositions suivantes:

| Emulsion nettoyante : | |
|---|---|
| Glycérine | 5,0 % |
| Gel de polyacrylamide | 2,0 % |
| Huile de Limnanthes | 1,0 % |
| Cocoate éthyl hexyl | 1,0 % |
| Cyclomethicone | 2,0 % |
| Ricin hydrogéné (40 OE) | 1,0 % |
| Parfum | 0,2 % |
| Conservateurs | 0,6 % |
| Eau | qsp 100 |

| Lotion : | |
|---|---|
| Glycérine | 5,0 % |
| Gel polyacrylamide | 1,7 % |
| Cyclométhicone | 2,0 % |
| Huile de Limnanthes | 0,5 % |
| Huile minérale | 2,5 % |
| Parfum | 0,3 % |
| Conservateurs | 0,5 % |
| Eau | qsp 100 |

| Crème : | |
|---|---|
| Acide stéarique (40 OE) | 2,5 % |
| Ethyl hexyl palmitate | 4,0 % |
| Propylène glycol dioctanoate | 4,0 % |
| Alcool stéarylique | 2,5 % |
| Huile de Limnanthes | 3,0 % |
| Glycol dibéhénate | 3,0 % |
| Cyclométhicone | 3,0 % |
| Polyméthiconol | 2,0% |
| Huile de soja hydrogénée | 3,5 % |
| Conservateurs | 0,5 % |
| Eau | qsp 100 |
| Glycérine | 5,0 % |
| Gel polyacrylamide | 3,0 % |
| Parfum | 0,3% |

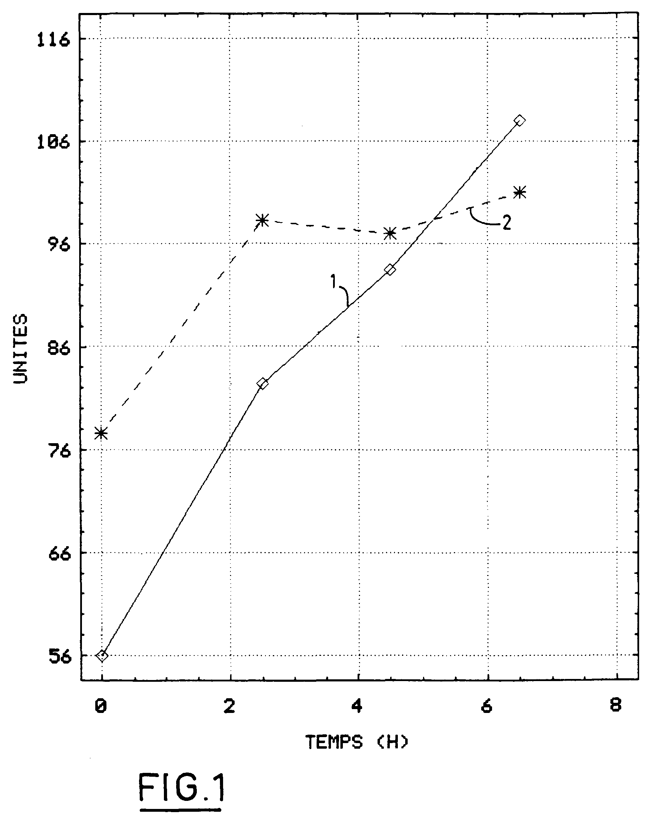

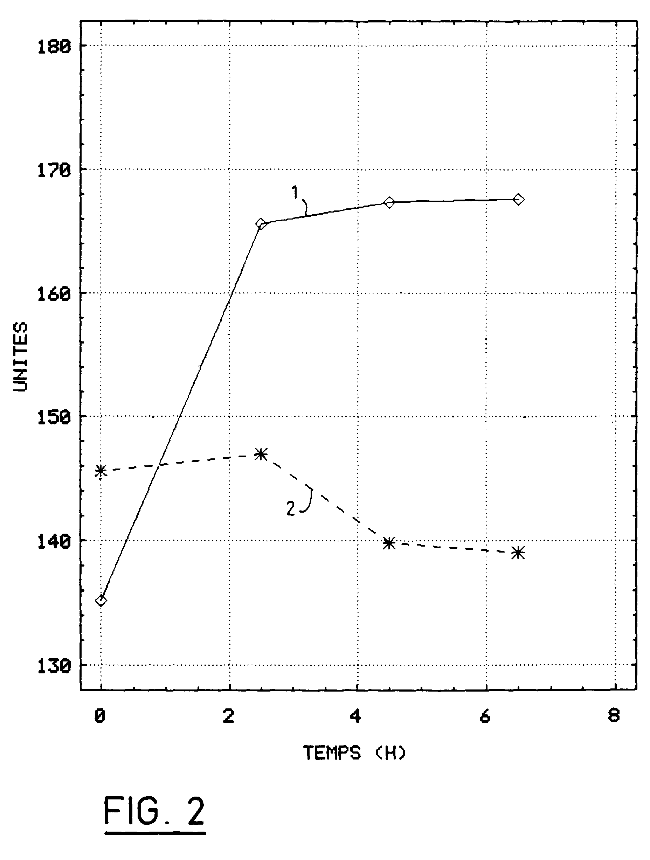

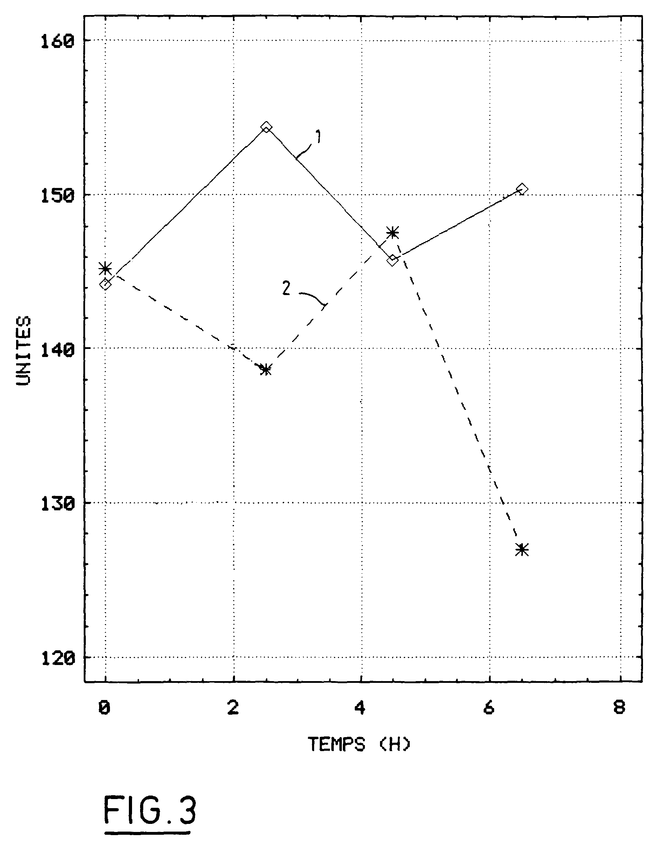

Les Figures 1, 2 et 3 montrent l'évolution du taux de sébum sur les différentes parties du visage (on mesure le sébum à l'aide d'un sébumètre, l'unité étant arbitraire). Plus spécifiquement, La Figure 1 représente l'évolution du taux de sébum sur les joues d'une personne présentant une peau mixte. La Figure 2 représente l'évolution du taux de sébum sur le nez d'une personne présentant une peau mixte. La Figure 3 représente l'évolution du taux de sébum sur le menton d'une personne présentant une peau mixte.

Sur chaque figure, la courbe 1 correspond à l'application d'une composition de référence ne comprenant pas la ou les substances actives de l'invention. De même, la courbe 2 correspond à l'application successive d'une émulsion nettoyante, d'une lotion et d'une crème tous les jours pendant deux semaines (y compris le jour où la mesure est effectuée).

### Résultats des figures 1, 2 et 3

La figure 1 montre que lorsqu'on applique sur les joues la composition de la présente invention, le taux de sébum est plus élevé au départ, puis augmente encore pour se stabiliser à un taux adéquat pour qu'une activité émolliente s'exerce sur la partie sèche de la peau. Par contre, l'autre essai montre un taux de sébum très faible au départ et très élevé après 6 heures.
La figure 2 montre que lorsqu'on applique sur le nez la composition de la présente invention, le taux de sébum se stabilise dans une gamme faible. L'autre essai de référence montre un taux de sébum qui augmente de façon très importante.
La figure 3 montre que lorsqu'on applique sur le menton la composition de la présente invention, le taux de sébum diminue après plus de 4 heures de façon importante. Par contre, l'essai de référence montre que le taux de sébum, après 4 heures, a tendance à augmenter.

Ainsi, les résultats indiquent avec la composition selon l'invention une nette augmentation du sébum au niveau des parties sèches (joues), et une nette diminution du sébum au niveau des parties grasses (nez, menton).

En plus de l'activité antilipase sur les zones grasses, les compositions cosmétiques de la présente invention ont donc une activité émolliente sur les zones sèches.

Le mode de réalisation de la crème est illustré ci-après. On chauffe la phase huileuse à 80/85° C en agitant. On chauffe séparément la phase aqueuse à 80/85° C en agitant. On ajoute alors la phase huileuse à la phase aqueuse sous vive agitation. On laisse ensuite refroidir en agitant lentement. Lorsque le mélange atteint une température voisine de 30° C, on ajoute le parfum.

Les quantités des substances actives efficaces sont fonction des types de formulation et des différents constituants choisis, sachant que l'efficacité du traitement réside dans les deux types d'activités antilipase et effet émollient, les quantités efficaces étant dosées afin que la composition ne présente pas un aspect trop gras, ne soit pas non plus irritable et bien entendu soit efficace pour le traitement des peaux mixtes.

| **EXEMPLE DE FORMULATION** | |
|---|---|
| **Crème protectrice :** | |
| Huile de Limnanthes | 12 % |
| Alcool cétylique | 2,0 % |
| Filtre UVB | 2,0% |
| Filtre UVA | 1,2% |
| Alcool laurique polyoxyéthyléné | 2,5 % |
| Stéarate de sorbitan | 2,5 % |
| Stéarate de sorbitan polyoxyéthyléné | 1,0 % |
| EdTA tétrasodique | 0,1 % |
| Carbopol | 0,35 % |
| Soude | 0,10 % |
| Parfum | 0,2 % |
| Conservateurs | 0,4 % |
| Eau | qsp 100 |

## Revendications

1. Utilisation, pour le traitement esthétique des peaux mixtes, d'une composition cosmétique comprenant une quantité efficace d'une substance choisie parmi l'huile de Limnanthes Alba et l'huile de Jessenia Bataua.

2. Utilisation selon la revendication 1, caractérisée en ce que la substance est l'huile de Limnanthes Alba.

3. Utilisation selon la revendication 2, caractérisée en ce que la composition contient de 0,5 à 15 % d'huile de Limnanthes Alba.

4. Utilisation selon la revendication 1, caractérisée en ce que la substance est l'huile de Jessenia Bataua.

5. Utilisation selon la revendication 4, caractérisée en ce que la composition contient de 0,5 à 15 % d'huile de Jessenia Bataua.

6. Utilisation selon l'une quelconque des revendications 1 à 5, caractérisée en ce que la composition est sous la forme d'une crème, d'un sérum ou d'une lotion.

## Claims

1. Usage, for the aesthetic treatment of mixed skin, of a cosmetic composition comprising an effective quantity of a substance chosen from the oil of Limnanthes and the oil of Jessenia Bataua.

2. Usage, according to claim 1, characterised in that the substance is the oil of Limnanthes Alba.

3. Usage according to claim 2, characterised in that the composition contains from 0.5 to 15% of the oil of Limnanthes Alba.

4. Usage according to claim 1, characterised in that the substance is the oil of Jessenia Bataua.

5. Usage according to claim 4, characterised in that, the composition contains from 0.5 to 15% of the oil of Jessenia Bataua.

6. Usage according to any one of the claims 1 to 5, characterised in that the composition is the form of a cream, of a serum, or of a lotion.

## Patentansprüche

1. Verwendung einer kosmetischen Zubereitung enthaltend eine wirksame Menge einer Substanz ausgewählt aus dem Öl von Limnanthes Alba und dem Öl von Jessenia Bataua zur ästhetischen Behandlung von Mischhaut.

2. Verwendung nach Anspruch 1, dadurch gekennzeichnet, daß die Substanz das Öl von Limnanthes Alba ist.

3. Verwendung nach Anspruch 2, dadurch gekennzeichnet, daß die Zubereitung 0,5 bis 15 % Öl von Limnanthes Alba enthält.

4. Verwendung nach Anspruch 1, dadurch gekennzeichnet, daß die Substanz das Öl von Jessenia Bataua ist.

5. Verwendung nach Anspruch 4, dadurch gekennzeichnet. daß die Zubereitung 0,5 bis 15 % des Öls von Jessenia Bataua enthält.

6. Verwendung nach einem der Ansprüche 1 bis 5, dadurch gekennzeichnet, daß die Zubereitung in Form einer Creme, eines Serums oder einer Lotion vorliegt.
